(19) **Europäisches Patentamt European Patent Office Office européen des brevets**

(11) **EP 4 697 025 A1**

(12) **EUROPEAN PATENT APPLICATION**
published in accordance with Art. 153(4) EPC

(43) Date of publication:
**18.02.2026 Bulletin 2026/08**

(21) Application number: **24788476.0**

(22) Date of filing: **07.03.2024**

(51) International Patent Classification (IPC):
*G01N 33/74* (2006.01)

(52) Cooperative Patent Classification (CPC):
**G01N 33/74**

(86) International application number:
**PCT/JP2024/008821**

(87) International publication number:
**WO 2024/214446 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.04.2023 JP 2023065013**

(71) Applicant: **Kobayashi, Hideyuki**
**Tokyo 143-8540 (JP)**

(72) Inventor: **Kobayashi, Hideyuki**
**Tokyo 143-8540 (JP)**

(74) Representative: **Lewis Silkin LLP**
**Arbor**
**255 Blackfriars Road**
**London SE1 9AX (GB)**

(54) **PREDICTION MODEL AND RISK DETERMINATION METHOD FOR MALE INFERTILITY**

(57)    This invention is intended to determine a risk of male infertility based on hormone levels in body fluid. The prediction model of the invention determines a risk of male infertility. The prediction model is a learned model prepared by performing machine learning using training data composed of a feature quantity including the follicle stimulating hormone level in the subject's body fluid and status information indicating the status of the subject's total motile sperm count, and the prediction model causes a computer to generate the status information indicating the status of the subject's total motile sperm count when the feature quantity including the follicle stimulating hormone level in the subject's body fluid is inputted.

Fig. 3

```
        Start
          │
          ▼
Blood sampling and age questioning
          │
          ▼
Measurement of hormone levels in blood
          │
          ▼
 Acquisition of feature quantity
          │
          ▼
  Input of feature quantity
          │
          ▼
Prediction of total motile sperm count status
          │
          ▼
  Output of prediction results
          │
          ▼
        End
```

## Description

### Technical Field

The present invention relates to a method for determining a risk of male infertility without using the total motile sperm count determined by the semen test.

### Background Art

In recent years, the trend toward delayed marriage is increasing due to women's participation in society, the diversification of lifestyles, and other reasons. According to the 2015 Population Survey Report, the average age for first marriage for males is 31.1 years and that for females is 29.4 years. That is, the average age for first marriage for males is risen by 2.6 years and that for females is risen by 3.1 years since 20 years ago. According to the survey of the same year, the average age at first birth for females is 30.7 years, which keeps increasing since records began. In general, female fertility, which is the ease of pregnancy, declines with age and late marriage is a cause of the low birthrate.

Meanwhile, the term "infertility" is defined as the inability to achieve pregnancy after a given period of time (e.g., a year) of regular unprotected intercourse between a man and a woman of reproductive ages with the intent to achieve pregnancy. According to a WHO survey, statistics show that roughly 50% of infertility cases are attributable to males.

In general, infertility attributable to males; i.e., male infertility, is diagnosed by the semen test and the urological test. The semen test comprises collecting a semen sample and measuring the semen volume, the sperm concentration, and the sperm motility. The semen test necessitates facilities or apparatuses, such as a collection room to obtain the total volume of a semen sample, the Makler sperm counting chamber, and a sperm counting apparatus, depending on the techniques to be adopted, and it is difficult to implement the semen test without such facilities or apparatuses.

In order to attain accurate test results, also, it is necessary to implement the semen test within a short period of time (several hours at longest) after the semen sample is obtained. In addition, males feel uncomfortable to take the semen test.

For example, Patent literature 1 discloses that mutation in a particular gene can be used for diagnosis of male infertility. However, genetic screening is not a simple test and a simple method to determine a risk of male infertility has not been known at present.

### Citation List

### Patent Literature

Patent Literature 1: JP 2022-542649 A

### Summary of Invention

### Technical Problem

As described above, a simple method for diagnosing male infertility or determining a risk of male infertility has not been known, and a simple method for diagnosing male infertility or determining a risk of male infertility without performing the semen test has been awaited. Under the above circumstances, it is an object of the present invention to provide a method for determining a risk of male infertility based on hormone levels in body fluid.

### Solution to Problem

In order to attain the object described above, the present inventor performed machine learning using the measurements of the hormone levels in body fluid and the results of the semen test obtained from a patient with male infertility. As a result, the present inventor discovered that a risk of male infertility could be determined based on the hormone levels in body fluid. This has led to the completion of the present invention.

The present invention includes the following.

(1) A prediction model configured to determine a risk of male infertility,

wherein the prediction model is a learned model prepared by performing machine learning using training data composed of a feature quantity including the follicle stimulating hormone level in the subject's body fluid and status information indicating the status of the subject's total motile sperm count, and
the prediction model causes a computer to generate (calculate) the status information indicating the status of the subject's total motile sperm count when the feature quantity including the follicle stimulating hormone level in the subject's body fluid is inputted.

(2) A method for determining a risk of male infertility using the prediction model described above comprising:

an acquisition step of measuring the follicle stimulating hormone level in the subject's body fluid to acquire a feature quantity including the follicle stimulating hormone level in the body fluid; and
a prediction step of predicting the status of the subject's total motile sperm count based on the feature quantity including the follicle stimulating hormone level in the body fluid using the prediction model.

The present description includes part or all of the contents as disclosed in the description and/or drawings of Japanese Patent Application No. 2023-65013, which is a priority document of the present application.

Advantageous Effects of Invention

The method for determining a risk of male infertility according to the present invention comprises measuring the follicle stimulating hormone level in body fluid and using the follicle stimulating hormone level in body fluid to determine a risk of male infertility. Specifically, the method for determining a risk of male infertility according to the present invention can be implemented in a simple manner unlike the semen test that necessitates special facilities or apparatuses.

Brief Description of Drawings

[Figure 1] Figure 1 schematically illustrates the apparatus for determining a risk of male infertility according to the first embodiment using the prediction model according to the first embodiment.
[Figure 2] Figure 2 schematically shows the constitution of a computer that realizes the apparatus for determining a risk of male infertility according to the first embodiment.
[Figure 3] Figure 3 shows a flow diagram demonstrating the method for determining a risk of male infertility according to the first embodiment.
[Figure 4] Figure 4 shows a flow diagram demonstrating the method for determining a risk of male infertility according to the second embodiment.
[Figure 5] Figure 5 shows a confusion matrix demonstrating the relationship between the actual measured value and the predicted value in binary classification problems.
[Figure 6] Figure 6 shows a confusion matrix and evaluation indexes obtained using the follicle stimulating hormone level in the blood in a blood sample as the training data (learning data) (threshold: 0.3).
[Figure 7] Figure 7 shows a confusion matrix and evaluation indexes obtained using the follicle stimulating hormone level in the blood and the luteinizing hormone level in the blood in a blood sample as the training data (threshold: 0.3).
[Figure 8] Figure 8 shows a confusion matrix and evaluation indexes obtained using the follicle stimulating hormone level in the blood and the testosterone level in the blood in a blood sample as the training data (threshold: 0.33).
[Figure 9] Figure 9 shows a confusion matrix and evaluation indexes obtained using the follicle stimulating hormone level in the blood, the luteinizing hormone level in the blood, and the testosterone level in the blood in a blood sample as the training data (threshold: 0.3).
[Figure 10A] Figure 10A shows a confusion matrix and evaluation indexes obtained by predicting normal (0) or abnormal (1) based on a feature quantity including the FSH level, the LH level, the T level, the E2 level, and the PRL level in the blood, the T:E2 ratio, and the patient's age.
[Figure 10B] Figure 10B shows a confusion matrix and evaluation indexes obtained by predicting normal (0) or abnormal (1) on the evaluation data for each of 88 cases of patients with azoospermia based on the similar feature quantity.
[Figure 11] Figure 11 shows a confusion matrix and evaluation indexes obtained by predicting normal (0) or abnormal (1) based on a feature quantity consisting of the FSH level in the blood.
[Figure 12] Figure 12 shows a confusion matrix and evaluation indexes obtained by predicting normal (0) or abnormal (1) based on a feature quantity including the FSH level and the LH level in the blood.
[Figure 13] Figure 13 shows a confusion matrix and evaluation indexes obtained by predicting normal (0) or abnormal (1) based on a feature quantity including the FSH level and the T level in the blood.
[Figure 14] Figure 14 shows a confusion matrix and evaluation indexes obtained by predicting normal (0) or abnormal (1) based on a feature quantity including the FSH level, the T level, and the LH level in the blood.

Description of Embodiments

At the outset, the outlines of the prediction model and the method for determining a risk of male infertility according to the embodiments of the present invention are described with reference to the first embodiment and the second embodiment.

<<First embodiment>>

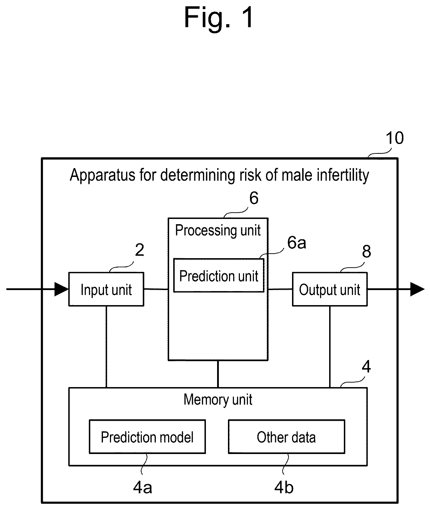

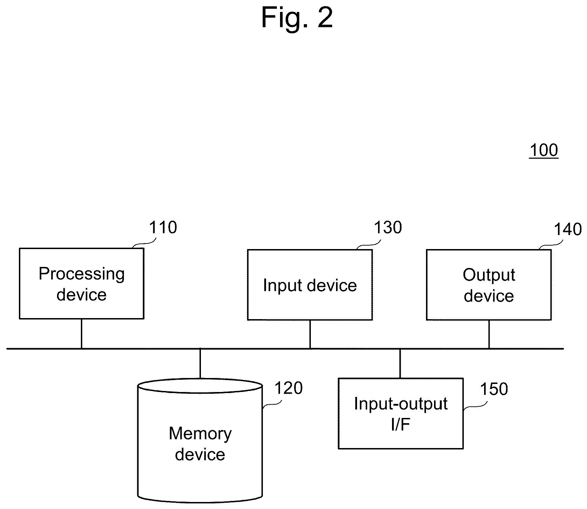

The prediction model according to the first embodiment is described as a prediction model used for the apparatus for determining a risk of male infertility according to the first embodiment. Figure 1 schematically illustrates the apparatus for determining a risk of male infertility according to the first embodiment using the prediction model according to the first embodiment. Figure 2 schematically shows the constitution of a computer that realizes the apparatus for determining a risk of male infertility according to the first embodiment.
As shown in Figure 1, the apparatus 10 for determining a risk of male infertility according to the first embodiment is equipped with the input unit 2, the memory unit 4, the processing unit 6 including the prediction unit 6a, and the output unit 8. The apparatus 10 for determining a risk is realized by, for example, the computer 100 shown in Figure 2. The computer 100 comprises the processing device (computing machine) 110, the memory device 120, the input device 130, the output device 140, the input-output interface (I/F) 150, and the like.

The input unit 2 of the apparatus 10 for determining a risk inputs a feature quantity of a subject of evaluation concerning a risk of male infertility. The feature quantity of the subject of evaluation includes the follicle stimulating hormone (FSH) level (FSH level), the luteinizing hormone (LH) level (LH level), the testosterone (T) level (T level), the estradiol (E2) level (E2 level), and the prolactin (PRL) level (PRL level) in the blood (body fluid) of the subject of evaluation, the T:E2 ratio, and the age of the subject of evaluation.

The memory unit 4 stores the prediction model 4a according to the first embodiment and other data 4b therein. The prediction model 4a determines a risk of male infertility. The prediction model 4a is a learned model prepared by performing machine learning using a plurality of training data sets obtained from each of a plurality of subjects of learning. The training data is a set of a feature quantity of a subject of learning and a classification label used in binary classification to indicate whether or not the total motile sperm count of the subject of learning is equivalent to or higher than the minimum of the normal range (e.g., $9.408 \times 10^6$) (normal (0): the total motile sperm count is equivalent to or higher than the minimum of the normal range; abnormal (1): the total motile sperm count is lower than the minimum of the normal range, the status information indicating the status of the subject's total motile sperm count). A feature quantity of a subject of learning includes the FSH level, the LH level, the T level, the E2 level, and the PRL level in the blood (body fluid) of the subject of learning, the T:E2 ratio, and the age of the subject of learning. The prediction model 4a causes the computer 100 to generate (calculate) the status information when the feature quantity is inputted.

The prediction unit 6a of the processing unit 6 uses the prediction model 4a to predict whether or not the total motile sperm count of a subject of evaluation is equivalent to or higher than the minimum of the normal range based on the feature quantity of the subject of evaluation inputted through the input unit 2. The output unit 8 outputs the results of prediction attained by the prediction unit 6a of the processing unit 6.

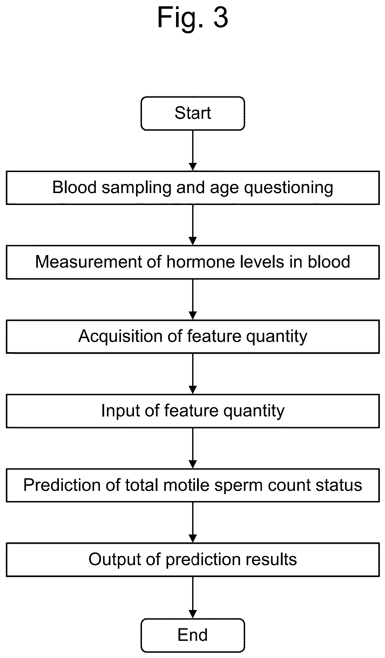

The method for determining a risk of male infertility according to the first embodiment is described. The method for determining a risk of male infertility according to the first embodiment comprises determining a risk of male infertility with the use of the prediction model 4a according to the first embodiment in the apparatus 10 for determining a risk according to the first embodiment. Figure 3 shows a flow diagram demonstrating the method for determining a risk of male infertility according to the first embodiment.

In the method for determining a risk of male infertility according to the first embodiment, as shown in Figure 3, a blood (body fluid) sample is obtained from a subject of evaluation concerning a risk of male infertility and the age is questioned in the beginning (blood sampling and age questioning). Subsequently, the FSH level, the LH level, the T level, the E2 level, and the PRL level in the blood sample are measured (measurement of hormone levels in blood). Subsequently, the T level is divided by the E2 level to determine the T:E2 ratio. Thus, a feature quantity including the FSH level, the LH level, the T level, the E2 level, and the PRL level in the blood sample, the T:E2 ratio, and the age questioned is acquired as the feature quantity of the subject of evaluation (acquisition of feature quantity).

Subsequently, a feature quantity of a subject of evaluation is inputted through the input unit 2 of the apparatus 10 for determining a risk (input of feature quantity). The inputted feature quantity is stored in the memory unit 4 by the processing unit 6. Subsequently, the prediction unit 6a of the processing unit 6 of the apparatus 10 for determining a risk uses the prediction model 4a to predict whether or not the total motile sperm count of the subject of evaluation is equivalent to or higher than the minimum of the normal range (e.g., $9.408 \times 10^6$) based on the feature quantity of the subject of evaluation (prediction of total motile sperm count status). Specifically, a feature quantity of a subject of evaluation is inputted into the computer 100, and the prediction model 4a causes the computer 100 to generate (calculate) a classification label that indicates whether or not the total motile sperm count of the subject of evaluation is equivalent to or higher than the minimum of the normal range.

Subsequently, the prediction results are outputted by the output unit 8 of the apparatus 10 for determining a risk (output of prediction results). In the manner described above, whether or not the subject has a risk of male infertility is determined based on the prediction results.

According to the prediction model and the method for determining a risk of male infertility according to the first embodiment, the hormone levels in the blood of the subject of evaluation, such as the FSH level, the LH level, the T level, the E2 level, and the PRL level, can be measured, and a risk of male infertility of the subject can then be determined based on the hormone levels and the age of the subject of evaluation. Accordingly, a method for determining a risk of male infertility can be performed in a simple manner unlike the semen test that requires the use of special facilities or apparatuses.

<<The second embodiment>>

The prediction model and the method for determining a risk of male infertility according to the second embodiment are described with a focus on the points different from the first embodiment.

The prediction model according to the second embodiment is used in the apparatus for determining a risk of male infertility according to the second embodiment. The apparatus for determining a risk of male infertility according to the second embodiment is the same as the apparatus for determining a risk of male infertility according to the first embodiment except for the prediction model. The prediction model according to the second embodiment determines a risk of male infertility, and it is a learned model prepared by performing machine learning using a plurality of sets of training data respectively obtained from a plurality of subjects of learning. The training data according to the second embodiment are composed of a feature quantity of a subject of learning and a binary classification label used to indicate whether or not the total motile sperm count of the subject of learning is equivalent to or higher than the minimum of the normal range (e.g., $9.408 \times 10^6$) (normal (0): the total motile sperm count is equivalent to or higher than the minimum of the normal range; abnormal (1): the total motile sperm count is lower than the minimum of the normal range, the status information indicating the status of the subject's total motile sperm count). Unlike the feature quantity of a subject of learning according to the first embodiment, the feature quantity of a subject of learning according to the second embodiment includes the FSH level, the LH level, the T level, the E2 level, and the PRL level in the saliva (body fluid) of a subject of learning, the T:E2 ratio, and the age of the subject of learning. The prediction model according to the second embodiment causes the computer to generate the status information when the feature quantity is inputted.

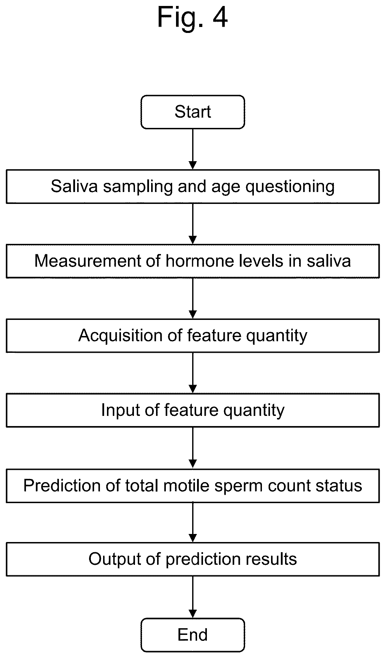

The method for determining a risk of male infertility according to the second embodiment comprises determining a risk of male infertility with the use of the prediction model according to the second embodiment in the apparatus for determining a risk according to the second embodiment. Figure 4 shows a flow diagram demonstrating the method for determining a risk of male infertility according to the second embodiment.

The method for determining a risk of male infertility according to the second embodiment is different from the first embodiment in that, as shown in Figure 4, a saliva (body fluid) sample is obtained from a subject of evaluation concerning a risk of male infertility and the age is questioned in the beginning (saliva sampling and age questioning). Subsequently, the FSH level, the LH level, the T level, the E2 level, and the PRL level in the saliva sample are measured (measurement of hormone levels in saliva). Subsequently, the T level is divided by the E2 level to determine the T:E2 ratio. Thus, a feature quantity including the FSH level, the LH level, the T level, the E2 level, and the PRL level in the saliva sample, the T:E2 ratio, and the age questioned is acquired as the feature quantity of the subject of evaluation (acquisition of feature quantity).

Subsequently, a feature quantity of a subject of evaluation is inputted through the input unit of the apparatus for determining a risk (input of feature quantity). The inputted feature quantity is stored in the memory unit by the processing unit. Subsequently, the prediction unit of the processing unit of the apparatus for determining a risk uses the prediction model to predict whether or not the total motile sperm count of the subject of evaluation is equivalent to or higher than the minimum of the normal range based on the feature quantity of the subject of evaluation (prediction of total motile sperm count status). Specifically, a feature quantity of a subject of evaluation is inputted into the computer 100, and the prediction model 4a causes the computer 100 to generate (calculate) a classification label that indicates whether or not the total motile sperm count of the subject of evaluation is equivalent to or higher than the minimum of the normal range.

Subsequently, the prediction results are outputted by the output unit of the apparatus for determining a risk (output of prediction result). In the manner described above, whether or not the subject has a risk of male infertility is determined based on the prediction results.

According to the prediction model and the method for determining a risk of male infertility according to the second embodiment, the hormone levels in the saliva sample of the subject of evaluation, such as the FSH level, the LH level, the T level, the E2 level, and the PRL level, can be measured, and a risk of male infertility of the subject can then be determined based on the hormone levels and the age of the subject of evaluation. Accordingly, a method for determining a risk of male infertility can be performed in a simple manner unlike the semen test that requires the use of special facilities or apparatuses. In addition, in comparison with the determination of a risk of male infertility of the subject by measuring the blood hormone levels of the subject of evaluation performed in the first embodiment, a risk can be determined by simply obtaining a saliva sample instead of obtaining a blood sample from the subject. Thus, a risk can be determined with lower invasiveness.

Subsequently, the prediction model and the method for determining a risk of male infertility according to the embodiments of the present invention are described in detail.

1. Prediction model

The prediction model configured to determine a risk of male infertility is a learned model prepared by performing machine learning using training data composed of a feature quantity including the follicle stimulating hormone (FSH) level (FSH level) in the subject's body fluid and the status information indicating the status of the subject's total motile sperm count.

The feature quantity (explanatory variable) in the training data is not particularly limited, provided that it includes the FSH level in the subject's body fluid. Examples thereof include a feature quantity further including at least one selected from the group consisting of the luteinizing hormone (LH) level (LH level), the testosterone (T) level (T level), the estradiol (E2) level (E2 level), and the prolactin (PRL) level (PRL level) in the subject's body fluid, the T:E2 ratio, and the subject's age. A feature quantity further including the LH level and/or the T level (at least one of the LH level or the T level) in the subject's body fluid, a feature quantity further including the E2 level in the subject's body fluid, a feature quantity further including the T:E2 ratio, and a feature quantity further including the subject's age are preferable. Among them, a feature quantity further including the LH level, the T level, and the E2 level in the subject's body fluid and the subject's age is further preferable, and a feature quantity further including the T:E2 ratio in addition to these is particularly preferable. The T:E2 ratio is determined by dividing the T level in the subject's body fluid by the E2 level in the subject's body fluid.

The follicle stimulating hormone is a glycoprotein having a molecular weight of approximately 33,000 and consisting of $\alpha$ and $\beta$ subunits. The luteinizing hormone is a glycoprotein having a molecular weight of approximately 29,000 and consisting of $\alpha$ and $\beta$ subunits. Testosterone is a steroid hormone represented by the molecular formula: $C_{19}H_{28}O_2$ and belonging to a group of androgens. An example thereof is total testosterone. Estradiol (follicle hormone (E2, an estrogen)) is a steroid hormone represented by the molecular formula: $C_{18}H_{24}O_2$. Prolactin is a hormone secreted by the pituitary gland.

Examples of body fluids in which the hormone levels of the hormones included in the feature quantity (FSH level, LH level, T level, E2 level, and PRL level) are to be measured include blood, saliva, and urine, and saliva and urine are preferable because invasiveness can further be lowered.

The blood of the subject (the blood sample) encompasses the whole blood, the serum, and the plasma. More specifically, the blood, serum, and plasma samples obtained can be used as in the case of the general peripheral blood test or biochemical test.

The follicle stimulating hormone level in the blood (FSH level (mIU/ml)) may be measured by any known method, and electrochemiluminescence immunoassay (ECLIA) that is generally used can be used herein. In addition to ECLIA, techniques involving the use of an antibody to the follicle stimulating hormone, such as the enzyme-labelled antibody technique, chemiluminescent enzyme immunoassay (CLEIA), chemiluminescent immunoassay (CLIA), enzyme-linked immunosorbent assay (ELISA), fluorescence enzyme immunoassay (FEIA), fluorescence immunoassay (FIA), latex agglutination (LA), latex agglutination (LA) turbidimetric immunoassay, and radio immunoassay (RIA), can adequately be used.

The hormone levels in the blood other than the follicle stimulating hormone (LH level (mIU/ml), T level (ng/ml), E2 level (pg/ml), and PRL level (ng/ml)) may be measured by any known method, and any of the techniques exemplified as the methods for measuring the follicle stimulating hormone described above can adequately be used.

The hormone levels in the subject' saliva (FSH level (mIU/ml), LH level (mIU/ml), T level (ng/ml), E2 level (pg/ml), and PRL level (ng/ml)) may be measured by any known method, and electrochemiluminescence immunoassay (ECLIA) that is generally used can be used herein. In addition to ECLIA, techniques involving the use of an antibody to the follicle stimulating hormone, such as the enzyme-labelled antibody technique, chemiluminescent enzyme immunoassay, chemiluminescent immunoassay, enzyme-linked immunosorbent assay, fluorescence enzyme immunoassay, fluorescence immunoassay, latex agglutination, latex agglutination turbidimetric immunoassay, and radio immunoassay, can adequately be used. Examples of apparatuses used for measuring hormone levels in the subject's saliva include the salivary EIA kit manufactured by Salimetrics LLC (e.g., the testosterone salivary immunoassay kit and the estradiol ELISA kit) and the expanded male hormone panel manufactured by DiagnosTechs, Inc.

The total motile sperm count of a subject is a numerical value obtained by multiplying the semen volume (mL), the sperm concentration (sperm/ml (sperm per milliliter)), and the sperm motility (%) of the subject (the semen volume $\times$ the sperm concentration $\times$ the sperm motility). The semen volume, the sperm concentration, and the sperm motility of the subject are measured by the subject's semen test. The status information (objective variable) indicating the status of the subject's total motile sperm count in the training data is not particularly limited, and the total motile sperm count of the subject itself may serve as the status information. For example, a classification label that indicates whether or not the total motile sperm count of the subject is equivalent to or higher than the minimum of the normal range (e.g., $9.408 \times 10^6$, the WHO laboratory manual for the examination and processing of human semen, 6th ed., 2021) is preferable as the status information.

A method for preparing a prediction model by performing machine learning using the training data is not particularly limited. Examples thereof include neural network, a support vector machine (SVM), decision tree regression, and random forest. Examples of software products and services for preparing a prediction model by performing machine learning using the training data include Prediction One (Sony Network Communications Inc.) and Google Cloud AutoML Tables (Google LLC).

The prediction model causes a computer to generate (calculate) the status information indicating the status of the subject's total motile sperm count when the feature quantity including the follicle stimulating hormone levels in the subject's body fluid is inputted. The prediction model is used in the prediction step in the method for determining a risk of male infertility according to the embodiment described below to predict the status of the total motile sperm count based on the feature quantity. The feature quantity inputted into the computer is the same as the feature quantity in the training data, and the description thereof is thus omitted herein. The status information generated (calculated) by the computer is the same as the status information in the training data, and the description thereof is thus omitted herein.

2. A method for determining a risk of male infertility

The method for determining a risk of male infertility using the prediction model according to the embodiments comprises: an acquisition step of measuring the follicle stimulating hormone (FSH) level (FSH level) in the subject's body fluid to acquire the feature quantity including the follicle stimulating hormone level in the body fluid; and a prediction step of predicting the status of the subject's total motile sperm count based on the feature quantity including the follicle stimulating hormone level in the body fluid using the prediction model.

The acquisition step is not particularly limited, provided that the step is as described above. The step of further measuring at least one hormone level selected from the group consisting of the luteinizing hormone level (LH level), the testosterone level (T level), the estradiol level (E2 level), and the prolactin level (PRL level) in the subject's body fluid is preferable as the acquisition step. The step of further measuring the LH level and/or T level (at least one of the LH level or the T level) in the subject's body fluid and the step of further measuring the E2 level in the subject's body fluid are more preferable as the acquisition step. The step of further measuring the LH level, the T level, and the E2 level in the subject's body fluid is particularly preferable as the acquisition step.

The feature quantity acquired in the acquisition step is the same as the feature quantity in the training data described in the "1. Prediction model" section above, and the description thereof is thus omitted herein. Examples of body fluids in which the hormone levels (FSH level, LH level, T level, E2 level, and PRL level) are measured in the acquisition step include the same body fluids as those described in the "1. Prediction model" section above. Examples of the method and the apparatus for measuring the hormone levels in body fluid used in the acquisition step are the same as those described in the "1. Prediction model" section above, and the description thereof is thus omitted herein.

The status of the subject's total motile sperm count predicted in the prediction step is not particularly limited. For example, it may be the status indicated by the status information in the training data described in the "1. Prediction model" section above. Specifically, the total motile sperm count of the subject itself may serve as the status. The label indicating whether or not the total motile sperm count of the subject is equivalent to or higher than the minimum of the normal range is preferable as the status.

As the method for determining a risk of male infertility, for example, the following determining method as in the case of the first embodiment and the second embodiment is preferable: a method for determining a risk of male infertility, wherein the type of body fluid in which the hormone level(s) included in the feature quantity in the training data used when preparing the prediction model is(are) measured is identical to the type of body fluid in which the hormone level(s) included in the feature quantity acquired in the acquisition step is(are) measured, and the type(s) of the hormone level(s) and the type(s) of other information(s) included in the feature quantity in the training data used when preparing the prediction model is(are) identical to the type(s) of the hormone level(s) and the type(s) of other information(s) included in the feature quantity acquired in the acquisition step. Such method is preferable since a risk of male infertility can be determined more accurately.

Alternatively, the method for determining a risk of male infertility may be a method for determining a risk of male infertility, wherein the type of body fluid in which the hormone level(s) included in the feature quantity in the training data used when preparing the prediction model is(are) measured is not identical to the type of body fluid in which the hormone level(s) included in the feature quantity acquired in the acquisition step is(are) measured, and the type(s) of the hormone level(s) and the type(s) of other information(s) included in the feature quantity in the training data used when preparing the prediction model is(are) identical to the type(s) of the hormone level(s) and the type(s) of other information(s) included in the feature quantity acquired in the acquisition step. Specifically, the method for determining a risk of male infertility may be a method for determining a risk of male infertility, wherein the prediction model is prepared by performing machine learning using the training data composed of a feature quantity including the hormone level(s) in the blood of the subject and the status information indicating the status of the subject's total motile sperm count, and the feature quantity acquired in the acquisition step includes the hormone level(s) of the same type(s) in the saliva instead of the blood of the subject. In such method of determination, the hormone level(s) in the saliva included in the feature quantity acquired in the acquisition step is(are) corrected to the approximate value(s) of the hormone level(s) in the blood based on the known correlation between the hormone level in the blood and the hormone level in the saliva, and the status of the total motile sperm count is then predicted based on the corrected feature quantity in the prediction step. The correlation between the hormone level in the blood and the hormone level in the saliva is described in, for example, S G Johnson et al., Direct assay for testosterone in saliva: relationship with a direct serum-free testosterone assay, Clin. Chim. Acta., 1987 Mar 30; 163 (3): 309-18, Jozef Vittek et al., Direct radioimmunoassay (ria) of salivary testosterone: correlation with free and total serum testosterone, Life Sci., Aug 26 1985; 37 (8): 711-6, and Shuhei Izawa et al., "The Comparison of Salivary Cortisol Immunoassay Kits" Japanese Journal of Complementary and Alternative Medicine 4(3) October 2007: 113-118.

In the method for determining a risk of male infertility, the type of the status information in the training data used when preparing the prediction model may or may not be identical to the type of the status predicted in the prediction step. In general, the type of the status information is identical to the type of the status. Specifically, when the type of the status information in the training data used when preparing the prediction model is the total motile sperm count of the subject, the type of the status predicted in the prediction step is the total motile sperm count of the subject. When the type of the status information in the training data used when preparing the prediction model is the classification label that indicates whether or not the total motile sperm count of the subject is equivalent to or higher than the minimum of the normal range, the type of the status predicted in the prediction step is the label indicating whether or not the total motile sperm count of the subject is equivalent to or higher than the minimum of the normal range.

3. Other methods for determining a risk of male infertility

The method for determining a risk of male infertility according to other embodiment comprises measuring the follicle stimulating hormone level in the blood sample of the subject and determining a risk of male infertility based on the follicle stimulating hormone level in the blood. While the examples below describe in detail that a risk of male infertility can be determined based on the follicle stimulating hormone level in the blood, it is realized as a result of construction of a machine learning model that predicts and analyzes the total motile sperm count (the semen volume × the sperm concentration × the sperm motility) based on the clinical data of the patient with male infertility. In other words, determination of a risk of male infertility in the method of the present embodiment is determination of a risk that the total motile sperm count of the subject is lower than the minimum of the normal range (e.g., $9.408 \times 10^6$, the WHO laboratory manual for the examination and processing of human semen, 6th ed., 2021).

In the method of the present embodiment, the follicle stimulating hormone level in the blood of the subject that is higher than the reference value indicates a risk of male infertility. In other words, the follicle stimulating hormone level in the blood of the subject that is higher than the reference value indicates a risk that the total motile sperm count may be lower than the minimum of the normal range. In the method of the present embodiment, the maximum (8.30 (mIU/ml) according to the test performed by SRL, Inc.) of the reference value in the usual blood test (2.00 to 8.30 (mIU/ml) according to the test performed by SRL, Inc.) may be adopted as the reference value of the follicle stimulating hormone level in the blood. A clinical reference value obtained from the clinical data may be adopted as the reference value of the follicle stimulating hormone level in the blood. According to the clinical data used in the examples below, the total motile sperm count would be equal to or lower than the minimum of the normal range when the follicle stimulating hormone level in the blood is higher than 8.81 (mIU/ml). When the follicle stimulating hormone level in the blood is lower than 4.00 (mIU/ml), the total motile sperm count is normal. Accordingly, the reference value of the follicle stimulating hormone level in the blood may be, for example, 4.00 (mIU/ml), 4.50 (mIU/ml), 5.00 (mIU/ml), 5.50 (mIU/ml), 6.00 (mIU/ml), 6.50 (mIU/ml), 7.00 (mIU/ml), 7.50 (mIU/ml), 8.00 (mIU/ml), 8.50 (mIU/ml), or 9.00 (mIU/ml).

In the method of the present embodiment, it is preferable that a subject be further subjected to measurement of the luteinizing hormone (LH) level and/or the testosterone (T) level, in addition to the follicle stimulating hormone level. In the method of the present embodiment, a risk of male infertility of the subject can be determined using the luteinizing hormone level and/or the testosterone level (at least one of the luteinizing hormone level or the testosterone level) in the blood (body fluid) in addition to the follicle stimulating hormone level in the blood of the subject.

In the method of the present embodiment, the subject is determined to have a risk of male infertility when the luteinizing hormone level in the blood of the subject is higher than the reference value in addition to the determination based on the follicle stimulating hormone level in the blood. In other words, the luteinizing hormone level in the blood of a subject that is higher than the reference value indicates a risk that the total motile sperm count may be lower than the minimum of the normal range. In the method of the present embodiment, a range of the reference value adopted in the general blood test (0.79 to 5.72 (mIU/ml) according to the test performed by SRL, Inc.) may be adopted as the reference range of the luteinizing hormone level in the blood. Alternatively, a clinical reference value obtained from the clinical data may be adopted as the range of the reference value. According to the clinical data used in the examples below, the luteinizing hormone level in the blood within the range of 3.10 to 4.70 (mIU/ml) indicates that the total motile sperm count would be normal, and the luteinizing hormone level in the bloods outside the range indicates that the total motile sperm count would be equal to or lower than the minimum of the normal range. Accordingly, the minimum of the range of the reference value of the luteinizing hormone level in the blood may be 0.85 (mIU/ml), 1.00 (mIU/ml), 1.50 (mIU/ml), 2.00 (mIU/ml), 2.50 (mIU/ml), 3.00 (mIU/ml), or 3.10 (mIU/ml), and the maximum of the range may be 5.50 (mIU/ml), 5.30 (mIU/ml), 5.00 (mIU/ml), or 4.70 (mIU/ml).

In the method of the present embodiment, in addition to the determination based on the follicle stimulating hormone level in the blood, the testosterone level in the blood of the subject that is lower than the reference value indicates a risk of male infertility. In other words, the testosterone level in the blood of the subject that is lower than the reference value indicates a risk that the total motile sperm count may be lower than the minimum of the normal range. In the method of the present embodiment, the minimum (1.31 (ng/ml) according to the test performed by SRL, Inc.) of the reference value in the usual blood test (1.31 to 8.71 (ng/ml) according to the test performed by SRL, Inc.) may be adopted as the reference value of the testosterone level in the blood. Alternatively, a clinical reference value obtained from the clinical data may be adopted as the range of the reference value. According to the clinical data used in the examples below, the total motile sperm count would be equal to or lower than the minimum of the normal range when the testosterone level in the blood is lower than 3.68 (ng/ml). When the testosterone level in the blood is higher than 4.59 (ng/ml), the total motile sperm count is normal. Accordingly, the reference value of the testosterone level in the blood may be, for example, 4.59 (ng/ml), 4.50 (ng/ml), 4.30 (ng/ml), 4.00 (ng/ml), 3.80 (ng/ml), or 3.70 (ng/ml).

In the method of the present embodiment, the luteinizing hormone level and/or the testosterone level (at least one of the luteinizing hormone level or the testosterone level) in the blood may be used in addition to the determination based on the follicle stimulating hormone level in the blood described above. This can further enhance the accuracy for determination of a risk of male infertility of the subject.

In the method of the present embodiment, the follicle stimulating hormone level in the blood, the luteinizing hormone level in the blood, and the testosterone level in the blood are compared with the relevant reference values respectively. Scores that are increased as the difference between each of these hormone levels and the relevant reference value increases may be calculated. A risk of male infertility may be determined in accordance with the total of the scores.

The blood sample used in the method of the present embodiment is the same as the blood (blood sample) described in the "1. Prediction model" section above, and the description thereof is thus omitted herein. The method for measuring the hormone levels (FSH level, LH level, and T level) in blood sample according to the method of the present embodiment is the same as the method for measuring the hormone levels in the blood described in the "1. Prediction model" section above, and the description thereof is thus omitted herein.

Examples

Hereafter, the present invention is described in greater detail with reference to the examples, although the technical scope of the present invention is not limited to the examples below.

[Example 1]

<Patient background>

The research described in the examples is performed in accordance with the authorization by the Ethics Committee of Toho University Omori Medical Center (authorization number: M22267 20104; research object: Cross-sectional clinical research on male infertility). In the present example, male patients at age 18 or over who had visited the Reproduction Center (Urology) in a period of time from January 1, 2011 to December 31, 2020 and underwent the semen test and the hormone test were designated as the targets in order to prepare AI models. The semen test was performed using the semen samples obtained via seminal emission in the hospital in all cases. The hormone test is based on the reference value defined by SRL, Inc. The data including the date of the first visit, the age, the luteinizing hormone (LH) (mIU/ml), the follicle stimulating hormone (FSH) (mIU/ml), the prolactin (PRL) (ng/ml), the total testosterone (T) (ng/ml), the estradiol (E2) (pg/ml), the semen volume (ml), the sperm concentration ($\times 10^6$/ml), and the motility (%) were extracted from the medical information (treatment information), and the prediction model creation (learning) data were prepared using Microsoft Excel®. As the data for verification of the AI models prepared in the example, in addition, the data including the date of the first visit, the age, the semen test, and the hormone test were extracted from the medical informations in a period of time from January 1, 2021 to December 31, 2021 and a period of time from January 1, 2022 to December 31, 2022, and the evaluation data were prepared as in the case of the prediction model creation (learning) data.

<Preparation of AI model that predicts male infertility>

The T:E2 ratio was calculated based on the T level and the E2 level included in the prediction model creation (learning) data and added to the prediction model creation (learning) data. The T:E2 ratio is reported to serve as a more accurate index of sperm quality or sexual libido in comparison with the testosterone by itself (Abhyankar, N. et al., F&S 106 (3): e239-e240, 2016). In addition, the total motile sperm count was calculated based on the semen volume, the sperm concentration, and the motility included in the prediction model creation (learning) data. The total motile sperm count is a numerical value calculated by multiplying the semen volume, the sperm concentration, and the motility. As the minimum of the normal range, the semen volume of 1.4 ml, the sperm concentration of $16 \times 10^6$/ml, and the motility of 42% defined in the WHO laboratory manual for the examination and processing of human semen, 6th ed., 2021 were adopted, and the minimum of the normal total motile sperm count was determined to be $9.408 \times 10^6$ ($1.4 \times 16 \times 10^6 \times 0.42$). The total motile sperm count higher than $9.408 \times 10^6$ was defined as "normal (0)," and the total motile sperm count lower than $9.408 \times 10^6$ was defined as "abnormal (1)."

In the end, the prediction model creation (learning) data including the age, LH, FSH, PRL, T (testosterone), E2, T:E2 ratio, the semen volume, the sperm concentration, the motility, the total motile sperm count, and normal (0)/abnormal (1) were prepared. The item "normal (0)/abnormal (1)" is the item to be predicted.

AI models were prepared using Prediction One (Sony Network Communications Inc., Tokyo, Japan). The prediction model creation (learning) data were used to prepare AI models. AI models were prepared in performing binary classification to predict normal (0) or abnormal (1) using Prediction One. Prediction One used a total of 3662 cases for AI model preparation: i.e., randomly selected 3296 cases as the training data (learning data); and other 366 cases as the evaluation data.

In addition, AI models were prepared in performing binary classification to predict normal (0) or abnormal (1) using Google Cloud AutoML Tables (Google LLC) using the data of all the 3662 cases.

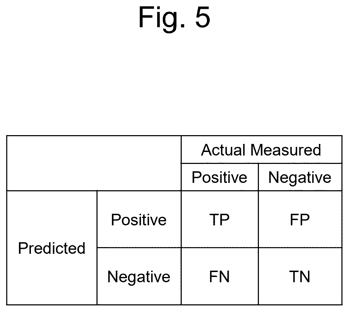

In general, the correlation between the actual measured value and the predicted value in binary classification can be demonstrated by the confusion matrix as shown in Figure 5. In the matrix, TP, FP, TN, and FN are as defined below.

TP (true positive): Both the actual measured value and the predicted value are positive values.
FP (false positive): The actual measured value is negative but is incorrectly predicted as positive (false positive).
TN (true negative): Both the actual measured value and the predicted value are negative values.
FN (false negative): The actual measured value is positive but is incorrectly predicted as negative (false negative).

Based on the TP, FP, TN, and FN levels, the evaluation indexes for prediction results; i.e., AUC (Area Under the Curve), Accuracy (Accuracy Rate, ACC), Precision, Recall, and F-measure, are determined. Accuracy is determined by the formula: (TP+TN)/(TP+TN+FP+FN). Precision is determined by the formula: (TP)/(TP+FP). Recall is determined by the formula: (TP)/(TP+FN). F-measure is the harmonic mean of precision and recall and it is determined by the formula: (2 Precision × Recall) / (Precision + Recall). AUC is determined as the area under the ROC curve drawn by plotting the true positive rates (TPR) on the vertical axis and the false positive rates (FRP) on the horizontal axis. TPR is the same as Recall and FPR is determined by the formula:

$$(FP)/(FP+TN).$$

<Results>

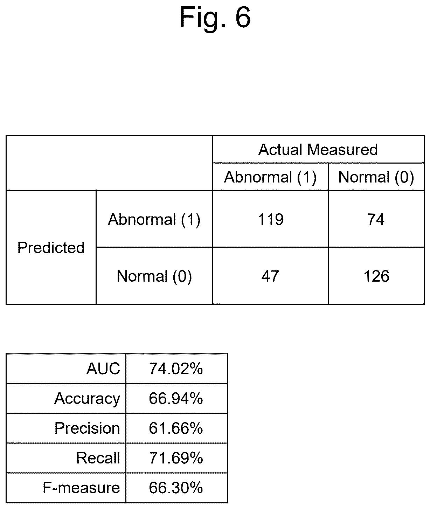

Figure 6 shows a confusion matrix obtained using the follicle stimulating hormone (FSH) level from the prediction model creation (learning) data as the training data and evaluation indexes. As shown in Figure 6, an AUC of 74.02% is achieved, and the model can be evaluated to yield excellent prediction accuracy. The results demonstrate that the follicle stimulating hormone (FSH) levels measured using blood samples can be used as the data to predict the total motile sperm count. Figure 6 shows a confusion matrix and the evaluation indexes at a threshold of 0.3.

Subsequently, variations of evaluation indexes occurring with the use of the data other than the follicle stimulating hormone (FSH) level were examined.

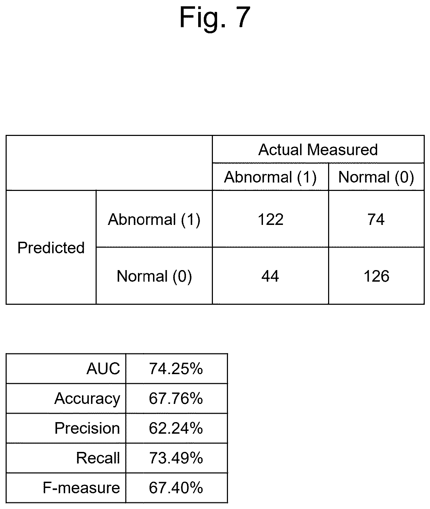

Figure 7 shows a confusion matrix and evaluation indexes obtained using the follicle stimulating hormone (FSH) level and the luteinizing hormone (LH) level from the prediction model creation (learning) data as the training data. As shown in Figure 7, an AUC of 74.25% is achieved, and the model can be evaluated to yield prediction accuracy superior to that achieved with the exclusive use of the follicle stimulating hormone (FSH) level. The results demonstrate that the follicle stimulating hormone (FSH) levels and the luteinizing hormone (LH) levels measured using blood samples can be used as the data to predict the total motile sperm count with higher accuracy. Figure 7 shows a confusion matrix and the evaluation indexes at a threshold of 0.3.

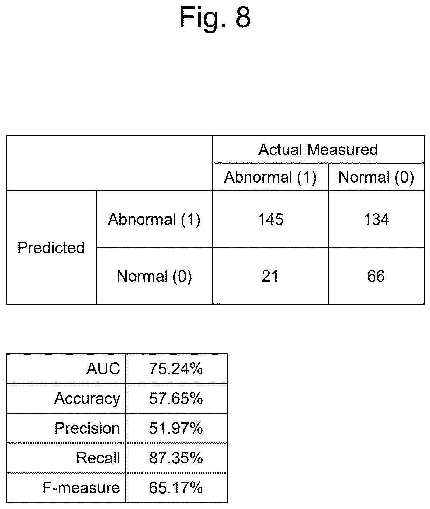

Figure 8 shows a confusion matrix and evaluation indexes obtained using the follicle stimulating hormone (FSH) level and the testosterone (T) level from the prediction model creation (learning) data as the training data. As shown in Figure 8, an AUC of 75.24% is achieved, and the model can be evaluated to yield prediction accuracy superior to the results demonstrated in Figure 6 and Figure 7. The results demonstrate that the follicle stimulating hormone (FSH) levels and the testosterone (T) levels measured using blood samples can be used as the data to predict the total motile sperm count with the highest accuracy. Figure 8 shows a confusion matrix and the evaluation indexes at a threshold of 0.33.

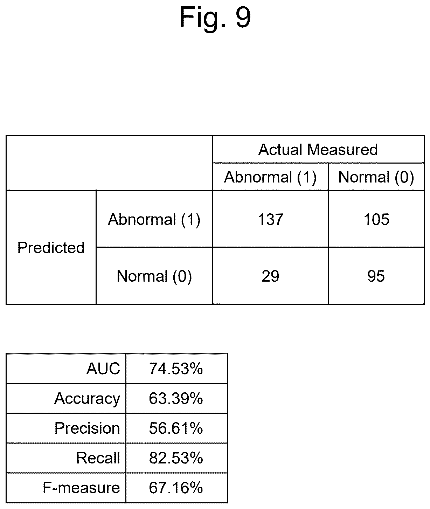

Figure 9 shows a confusion matrix and evaluation indexes obtained using the follicle stimulating hormone (FSH) level, the luteinizing hormone (LH) level, and the testosterone (T) level from the prediction model creation (learning) data as the training data. As shown in Figure 9, an AUC of 74.53% is achieved. While the prediction accuracy is lower than the results shown in Figure 8, the model can be evaluated to yield prediction accuracy superior to the results demonstrated in Figure 6 and Figure 7. The results demonstrate that the follicle stimulating hormone (FSH) levels, the luteinizing hormone (LH) levels, and the testosterone (T) levels measured using blood samples can be used as the data to predict the total motile sperm count with higher accuracy. Figure 9 shows a confusion matrix at a threshold of 0.3 and the evaluation indexes.

[Example 2]

The method for determining a risk of male infertility according to the embodiment was performed on the evaluation data of 354 cases different from the evaluation data of the 366 cases described above. The method is described in detail below.

From the medical information of 354 male patients (subjects) at age 18 or over who had visited the Reproduction Center (Urology) in a period of time from January 1, 2021 to December 31, 2022 and underwent the semen test and the hormone test (blood test), the data including the FSH level (mIU/ml), the LH level (mIU/ml), the T level (total testosterone level) (ng/ml), the E2 level (pg/ml), and the PRL level (ng/ml) in the blood, the age, the semen volume (ml), the sperm concentration ($\times$ $10^6$/ml), and the motility (%) were extracted. The T:E2 ratio was calculated based on the T level and the E2 level in the extracted data. When the total motile sperm count calculated based on the semen volume, the sperm concentration, and the motility, and the total motile sperm count was equivalent to or higher than the minimum of the normal range ($9.408 \times 10^6$), the classification label of binary classification was assigned a value of normal (0). When the total motile sperm count was lower than the minimum of the normal range, the classification label of binary classification was assigned a value of abnormal (1). The T:E2 ratio, the total motile sperm count, and the classification labels were then added to the extracted data of each subject to obtain the evaluation data of each subject. The evaluation data of 354 subjects was thus prepared. The evaluation data of each subject includes the FSH level, the LH level, the T level, the E2 level, and the PRL level in the patient's blood, the T:E2 ratio, and the age of the patient (the information constituting the feature quantity), and the semen volume, the sperm concentration, the motility, the total motile sperm count, and classification label (normal (0): the total motile sperm count is equivalent to or higher than the minimum of the normal range; abnormal (1): the total motile sperm count is lower than the minimum of the normal range).

The prediction model was prepared in the same manner as the preparation of the prediction model (AI model) of Example 1 by machine learning using Prediction One using the prediction model creation data (the learning data of 3296 subjects) as the training data. Thus, a prediction model that would cause the computer to generate the classification label to indicate whether or not the total motile sperm count of the patient would be equivalent to or higher than the minimum of the normal range upon input of the feature quantity in the evaluation data was prepared.

The results of determination of a risk of male infertility on the evaluation data of each of 354 subjects are described. With the use of the prediction model, whether or not the total motile sperm count of the patient would be equivalent to or higher than the minimum of the normal range (normal (0) or abnormal (1)) was predicted based on the feature quantity in the evaluation data of each subject. Specifically, a threshold of the score used to determine whether or not the total motile sperm count of the patient is equivalent to or higher than the minimum of the normal range was assigned each value, and the feature quantity in the evaluation data of each subject was inputted into the computer to generate the classification label. The predicted value obtained based on the evaluation data of each of 354 subjects (the generated classification label) was compared with the actual measured value (the classification label of the evaluation data) to determine Accuracy (Accuracy Rate, ACC), Precision, Recall, and F-measure.

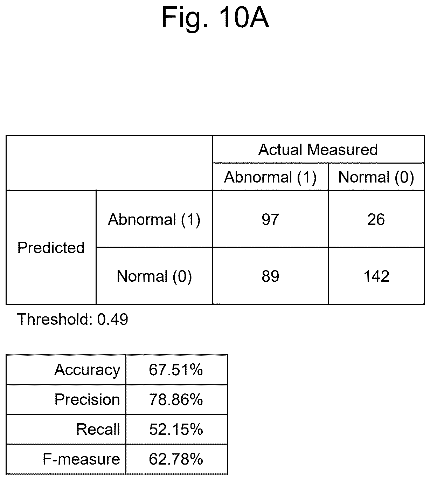

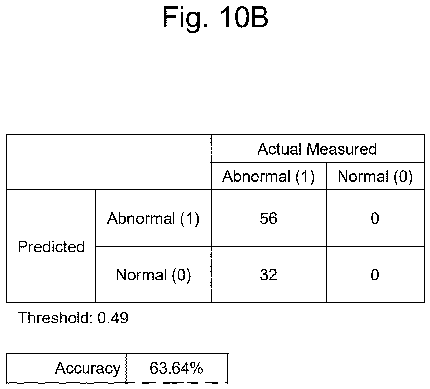

With the use of the prediction model prepared by performing machine learning using the training data composed of the feature quantity including the FSH level, the LH level, the T level, the E2 level, and the PRL level in the blood of the patient, the T:E2 ratio, and the age of the patient in the learning data and the classification label (normal (0) or abnormal (1)) to indicate whether or not the total motile sperm count of the patient in the learning data would be equivalent to or higher than the minimum of the normal range, the evaluation data of each of 354 subjects was subjected to prediction as to whether or not the total motile sperm count of the patient would be equivalent to or higher than the minimum of the normal range (normal (0) or abnormal (1)) based on the same feature quantity. In this case, the prepared prediction model yielded AUC of 74.42%. The prediction results achieved the accuracy of 67.51% when the threshold was 0.49 for maximizing the F-measure. Figure 10A shows a confusion matrix and evaluation indexes at a threshold of 0.49 in the prediction results. With the use of the same prediction model, the evaluation data of each of the 88 subjects with azoospermia (35 subjects: obstructive azoospermia; 52 subjects: non-obstructive azoospermia; 1 subject: hypogonadotropic hypogonadism) selected from the 354 subjects was subjected to prediction of normal (0) or abnormal (1) based on the same feature quantity. Figure 10B shows a confusion matrix and evaluation indexes at a threshold of 0.49 in the prediction results. When the threshold in the prediction results was 0.49, accuracy of 100% was achieved for 52 cases of non-obstructive azoospermia.

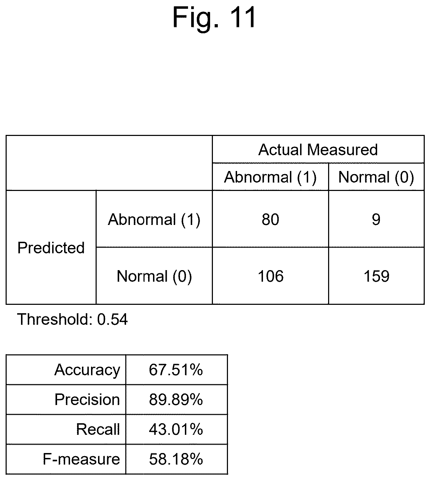

Subsequently, the prediction model prepared by performing machine learning using the training data composed of the feature quantity consisting of the FSH level in the blood of the patient in the learning data and the classification label (normal (0) or abnormal (1)) to indicate whether or not the total motile sperm count of the patient in the leaning data would be equivalent to or higher than the minimum of the normal range was used to predict whether the evaluation data of each of 354 subjects would be categorized as normal (0) or abnormal (1) based on the same feature quantity. In this case, the prepared prediction model yielded AUC of 74.02%. The prediction results achieved the accuracy of 67.51% when the threshold was 0.54 for maximizing the F-measure. Figure 11 shows a confusion matrix and evaluation indexes at a threshold of 0.54 in the prediction results.

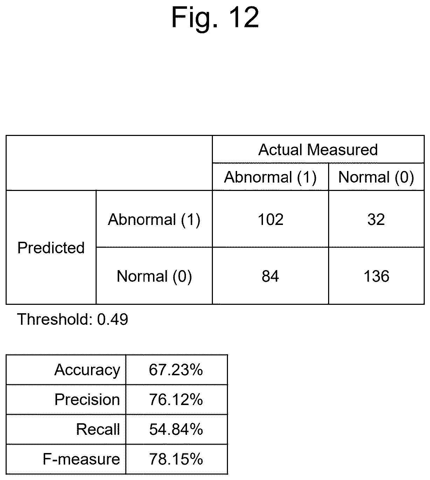

Subsequently, the prediction model prepared by performing machine learning using the training data composed of the feature quantity including the FSH level and the LH level in the blood of the patient in the leaning data and the classification label (normal (0) or abnormal (1)) to indicate whether or not the total motile sperm count of the patient in the leaning data would be equivalent to or higher than the minimum of the normal range was used to predict whether the evaluation data of each of 354 subjects would be categorized as normal (0) or abnormal (1) based on the same feature quantity. In this case, the prepared prediction model yielded AUC of 74.25%. The prediction results achieved the accuracy of 67.23% when the threshold was 0.49 for maximizing the F-measure. Figure 12 shows a confusion matrix and evaluation indexes at a threshold of 0.49 in the prediction results.

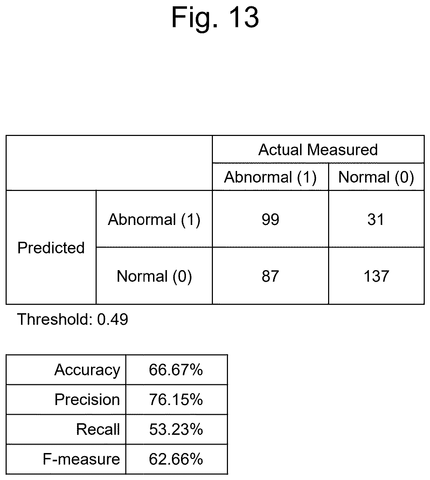

Subsequently, the prediction model prepared by performing machine learning using the training data composed of the feature quantity including the FSH level and the T level in the blood of the patient in the leaning data and the classification label (normal (0) or abnormal (1)) to indicate whether or not the total motile sperm count of the patient in the leaning data would be equivalent to or higher than the minimum of the normal range was used to predict whether the evaluation data of each of 354 subjects would be categorized as normal (0) or abnormal (1) based on the same feature quantity. In this case, the prepared prediction model yielded AUC of 75.24%. The prediction results achieved the accuracy of 66.67% when the threshold was 0.49 for maximizing the F-measure. Figure 13 shows a confusion matrix and evaluation indexes at a threshold of 0.49 in the prediction results.

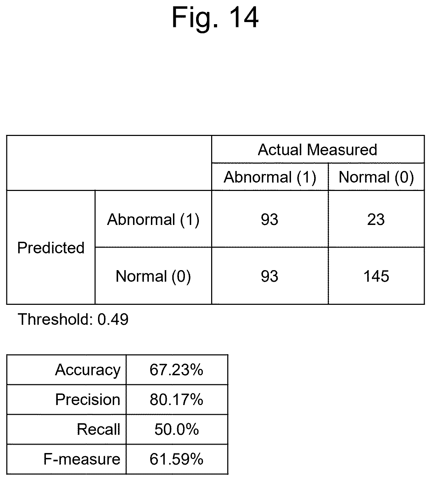

Subsequently, the prediction model prepared by performing machine learning using the training data composed of the feature quantity including the FSH level, the T level, and the LH level in the blood of the patient in the leaning data and the classification label (normal (0) or abnormal (1)) to indicate whether or not the total motile sperm count of the patient in the leaning data would be equivalent to or higher than the minimum of the normal range was used to predict whether the evaluation data of each of 354 subjects would be categorized as normal (0) or abnormal (1) based on the same feature quantity. In this case, the prepared prediction model yielded AUC of 74.53%. The prediction results achieved the accuracy of 67.23% when the threshold was 0.49 for maximizing the F-measure. Figure 14 shows a confusion matrix and evaluation indexes at a threshold of 0.49 in the prediction results.

The present invention includes the embodiments described below.

(1) A method for determining a risk of male infertility comprising a step of measuring the follicle stimulating hormone (FSH) level in a blood sample obtained from a subject, wherein the follicle stimulating hormone level in the blood indicates a risk of male infertility of the subject.
(2) The method according to (1) further comprising measuring the luteinizing hormone (LH) level and/or the testosterone (T) level in the blood sample, wherein the luteinizing hormone level in the blood and/or the testosterone level in the blood (at least one of the luteinizing hormone level in the blood or the testosterone level in the blood) indicates a risk of male infertility of the subject.
(3) The method according to (1), wherein the follicle stimulating hormone level in the blood which is higher than 4.00 (mIU/ml) indicates a risk of male infertility.
(4) The method according to (2), wherein the luteinizing hormone level in the blood which is outside of a range of 3.10 to 4.70 (mIU/ml) indicates a risk of male infertility.
(5) The method according to (2), wherein the testosterone level in the blood which is lower than 4.59 (ng/ml) indicates a risk of male infertility.

While the embodiments of the prediction model and the method for determining a risk of male infertility according to the present invention are described above in detail, the present invention is not limited to the embodiments described above, and various changes of design can be made without departing from the spirit of the present invention described in claims.

Reference Signs List

10: Apparatus for determining a risk of male infertility
2: Input unit
4: Memory unit
4a: Prediction model
6: Processing unit
6a: Prediction unit
8: Output unit
100: Computer
110: Processing device (calculator)
120: Memory device
130: Input device
140: Output device
150: Input-output interface (I/F)

All publications, patents, and patent applications cited herein are incorporated herein by reference in their entirety.

## Claims

1. A prediction model configured to determine a risk of male infertility,

   wherein the prediction model is a learned model prepared by performing machine learning using training data composed of a feature quantity including the follicle stimulating hormone level in the subject's body fluid and status information indicating the status of the subject's total motile sperm count, and

the prediction model causes a computer to generate the status information indicating the status of the subject's total motile sperm count when the feature quantity including the follicle stimulating hormone level in the subject's body fluid is inputted.

2. The prediction model according to claim 1, wherein the body fluid is blood.

3. The prediction model according to claim 1, wherein the body fluid is saliva.

4. The prediction model according to any one of claims 1 to 3, wherein the feature quantity further includes the luteinizing hormone level and/or the testosterone level in the body fluid.

5. A method for determining a risk of male infertility using the prediction model according to claim 1 comprising:

measuring the follicle stimulating hormone level in the subject's body fluid to acquire a feature quantity including the follicle stimulating hormone level in the body fluid; and
predicting the status of the subject's total motile sperm count based on the feature quantity including the follicle stimulating hormone level in the body fluid using the prediction model.

6. The method for determining a risk of male infertility according to claim 5, wherein the body fluid is blood.

7. The method for determining a risk of male infertility according to claim 5, wherein the body fluid is saliva.

8. The method for determining a risk of male infertility according to any one of claims 5 to 7, further measuring the luteinizing hormone level and/or the testosterone level in the body fluid in the measuring of the follicle stimulating hormone level and the feature quantity further includes the luteinizing hormone level and/or the testosterone level in the body fluid.

# Fig. 1

# Fig. 2

100

```
┌──────────────┐        ┌──────────────┐      ┌──────────────┐
│ 110          │        │ 130          │      │ 140          │
│ Processing   │        │ Input device │      │ Output       │
│ device       │        │              │      │ device       │
└──────┬───────┘        └──────┬───────┘      └──────┬───────┘
       │                       │                     │
───────┴───────────┬───────────┴──────────┬──────────┴────────
                   │                       │
            ┌──────┴──────┐ 120     ┌───────┴──────┐ 150
            │  Memory     │         │ Input-output │
            │  device     │         │ I/F          │
            └─────────────┘         └──────────────┘
```

# Fig. 3

```
            ┌──────────────┐
            │    Start      │
            └──────┬───────┘
                   │
                   ▼
┌──────────────────────────────────────────┐
│   Blood sampling and age questioning       │
└──────────────────┬───────────────────────┘
                   │
                   ▼
┌──────────────────────────────────────────┐
│   Measurement of hormone levels in blood   │
└──────────────────┬───────────────────────┘
                   │
                   ▼
┌──────────────────────────────────────────┐
│        Acquisition of feature quantity     │
└──────────────────┬───────────────────────┘
                   │
                   ▼
┌──────────────────────────────────────────┐
│           Input of feature quantity        │
└──────────────────┬───────────────────────┘
                   │
                   ▼
┌──────────────────────────────────────────┐
│  Prediction of total motile sperm count status │
└──────────────────┬───────────────────────┘
                   │
                   ▼
┌──────────────────────────────────────────┐
│         Output of prediction results       │
└──────────────────┬───────────────────────┘
                   │
                   ▼
            ┌──────────────┐
            │     End       │
            └──────────────┘
```

# Fig. 4

```
            ┌──────────────┐
            │    Start     │
            └──────┬───────┘
                   │
                   ▼
   ┌───────────────────────────────────┐
   │ Saliva sampling and age questioning│
   └───────────────┬───────────────────┘
                   │
                   ▼
   ┌───────────────────────────────────┐
   │ Measurement of hormone levels in saliva│
   └───────────────┬───────────────────┘
                   │
                   ▼
   ┌───────────────────────────────────┐
   │    Acquisition of feature quantity  │
   └───────────────┬───────────────────┘
                   │
                   ▼
   ┌───────────────────────────────────┐
   │      Input of feature quantity      │
   └───────────────┬───────────────────┘
                   │
                   ▼
   ┌───────────────────────────────────┐
   │ Prediction of total motile sperm count status│
   └───────────────┬───────────────────┘
                   │
                   ▼
   ┌───────────────────────────────────┐
   │     Output of prediction results    │
   └───────────────┬───────────────────┘
                   │
                   ▼
            ┌──────────────┐
            │     End      │
            └──────────────┘
```

# Fig. 5

|  |  | Actual Measured | |
|---|---|---|---|
|  |  | Positive | Negative |
| Predicted | Positive | TP | FP |
| | Negative | FN | TN |

# Fig. 6

| | | Actual Measured | |
|---|---|---|---|
| | | Abnormal (1) | Normal (0) |
| Predicted | Abnormal (1) | 119 | 74 |
| | Normal (0) | 47 | 126 |

| | |
|---|---|
| AUC | 74.02% |
| Accuracy | 66.94% |
| Precision | 61.66% |
| Recall | 71.69% |
| F-measure | 66.30% |

# Fig. 7

| | | Actual Measured | |
|---|---|---|---|
| | | Abnormal (1) | Normal (0) |
| Predicted | Abnormal (1) | 122 | 74 |
| | Normal (0) | 44 | 126 |

| | |
|---|---|
| AUC | 74.25% |
| Accuracy | 67.76% |
| Precision | 62.24% |
| Recall | 73.49% |
| F-measure | 67.40% |

# Fig. 8

|  |  | Actual Measured | |
|---|---|---|---|
|  |  | Abnormal (1) | Normal (0) |
| Predicted | Abnormal (1) | 145 | 134 |
| | Normal (0) | 21 | 66 |

| | |
|---|---|
| AUC | 75.24% |
| Accuracy | 57.65% |
| Precision | 51.97% |
| Recall | 87.35% |
| F-measure | 65.17% |

# Fig. 9

|  |  | Actual Measured | |
|---|---|---|---|
|  |  | Abnormal (1) | Normal (0) |
| Predicted | Abnormal (1) | 137 | 105 |
| | Normal (0) | 29 | 95 |

| | |
|---|---|
| AUC | 74.53% |
| Accuracy | 63.39% |
| Precision | 56.61% |
| Recall | 82.53% |
| F-measure | 67.16% |

# Fig. 10A

|  |  | Actual Measured | |
|---|---|---|---|
|  |  | Abnormal (1) | Normal (0) |
| Predicted | Abnormal (1) | 97 | 26 |
|  | Normal (0) | 89 | 142 |

Threshold: 0.49

| | |
|---|---|
| Accuracy | 67.51% |
| Precision | 78.86% |
| Recall | 52.15% |
| F-measure | 62.78% |

# Fig. 10B

| | | Actual Measured | |
|---|---|---|---|
| | | Abnormal (1) | Normal (0) |
| Predicted | Abnormal (1) | 56 | 0 |
| | Normal (0) | 32 | 0 |

Threshold: 0.49

| Accuracy | 63.64% |
|---|---|

# Fig. 11

|  |  | Actual Measured | |
|---|---|---|---|
|  |  | Abnormal (1) | Normal (0) |
| Predicted | Abnormal (1) | 80 | 9 |
|  | Normal (0) | 106 | 159 |

Threshold: 0.54

| Accuracy | 67.51% |
|---|---|
| Precision | 89.89% |
| Recall | 43.01% |
| F-measure | 58.18% |

# Fig. 12

|  |  | Actual Measured | |
|---|---|---|---|
|  |  | Abnormal (1) | Normal (0) |
| Predicted | Abnormal (1) | 102 | 32 |
|  | Normal (0) | 84 | 136 |

Threshold: 0.49

| Accuracy | 67.23% |
|---|---|
| Precision | 76.12% |
| Recall | 54.84% |
| F-measure | 78.15% |

# Fig. 13

|  |  | Actual Measured | |
|---|---|---|---|
|  |  | Abnormal (1) | Normal (0) |
| Predicted | Abnormal (1) | 99 | 31 |
|  | Normal (0) | 87 | 137 |

Threshold: 0.49

| Accuracy | 66.67% |
|---|---|
| Precision | 76.15% |
| Recall | 53.23% |
| F-measure | 62.66% |

# Fig. 14

| | | Actual Measured | |
|---|---|---|---|
| | | Abnormal (1) | Normal (0) |
| Predicted | Abnormal (1) | 93 | 23 |
| | Normal (0) | 93 | 145 |

Threshold: 0.49

| | |
|---|---|
| Accuracy | 67.23% |
| Precision | 80.17% |
| Recall | 50.0% |
| F-measure | 61.59% |

## INTERNATIONAL SEARCH REPORT

| International application No. |
| --- |
| **PCT/JP2024/008821** |

### A. CLASSIFICATION OF SUBJECT MATTER

*G01N 33/74*(2006.01)i
FI: G01N33/74

According to International Patent Classification (IPC) or to both national classification and IPC

### B. FIELDS SEARCHED

Minimum documentation searched (classification system followed by classification symbols)

G01N33/74

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
| --- | --- | --- |
| Y | HENRIKE, Krenz et al., Machine learning based prediction models in male reproductive health:Development of a proof-of-concept model for Klinefelter Syndrome in azoospermic patients, Andrology, 2022, Vol.10, no. 3, 534-544, <https://onlinelibrary.wiley.com/doi/epdf/10.1111/andr.13141>, <DOI: 10.1111/andri.13141> Material and methods, Results, table 2 | 1-8 |
| Y | WEI, Zhao et al., Circulating sex hormone levels in relation to male sperm quality, BMC Urology, 2020, vol. 20, no. 1, 1-7, <https://doi.org/10.1186/s12894-020-00674-7> Result, table 3 | 1-8 |
| A | JP 2018-513983 A (BLUDIAGNOSTICS, INC.) 31 May 2018 (2018-05-31) paragraph [0086] | 1-8 |
| A | JP 2007-24822 A (ASKA PHARMACEUTICAL CO., LTD.) 01 February 2007 (2007-02-01) paragraphs [0020]-[0025] | 1-8 |

☐ Further documents are listed in the continuation of Box C.   ☑ See patent family annex.

| | |
| --- | --- |
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **17 April 2024** | **07 May 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/008821**

| Patent document cited in search report | | | Publication date (day/month/year) | Patent family member(s) | | | Publication date (day/month/year) |
|---|---|---|---|---|---|---|---|
| JP | 2018-513983 | A | 31 May 2018 | WO | 2016/164365 | A1 | |
| | | | | paragraph [0088] | | | |
| | | | | US | 2018/0106799 | A1 | |
| | | | | EP | 3281014 | A1 | |
| JP | 2007-24822 | A | 01 February 2007 | (Family: none) | | | |

Form PCT/ISA/210 (patent family annex) (July 2022)

# EP 4 697 025 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2022542649 A

- JP 2023065013 A

### Non-patent literature cited in the description

- **JOHNSON et al.** Direct assay for testosterone in saliva: relationship with a direct serum-free testosterone assay. *Clin. Chim. Acta.*, 30 March 1987, vol. 163 (3), 309-18
- **JOZEF VITTEK et al.** Direct radioimmunoassay (ria) of salivary testosterone: correlation with free and total serum testosterone. *Life Sci.*, 26 August 1985, vol. 37 (8), 711-6

- **SHUHEI IZAWA et al.** The Comparison of Salivary Cortisol Immunoassay Kits. *Japanese Journal of Complementary and Alternative Medicine*, October 2007, vol. 4 (3), 113-118
- **ABHYANKAR, N. et al.** *F&S*, 2016, vol. 106 (3), e239-e240